# EUROPEAN PATENT SPECIFICATION

(11) **EP 4 032 491 B1**
(45) Date of publication and mention of the grant of the patent: **20.08.2025**
(21) Application number: 22157372.8
(22) Date of filing: 14.03.2014
(51) Int. Cl.: A61B 18/12, A61B 17/22

(54) **ELECTROSURGICAL DEVICE HAVING A LUMEN**
ELEKTROCHIRURGISCHE VORRICHTUNG MIT EINEM LUMEN
DISPOSITIF ÉLECTROCHIRURGICAL DOTÉ D'UNE LUMIÈRE

(30) Priority: 14.03.2013 US 201361781231 P
(43) Date of publication of application: 27.07.2022
(62) Divisional of application: 14764721.8
(73) Proprietor: Boston Scientific Medical Device Limited, Galway H91 Y868 (IE)
(72) Inventor: DAVIES, Gareth, Toronto, M5R 2N5 (CA); URBANSKI, John Paul, Toronto, M4L 2N7 (CA); ABOU-MARIE, Rund, Ontario, L5N 7S5 (CA)
(74) Representative: Pfenning, Meinig & Partner mbB

(56) References cited:
- EP-A1- 2 204 134
- EP-A1- 2 968 846
- WO-A1-03/082134
- WO-A2-2007/090075
- US-A1- 2002 111 620
- US-A1- 2011 118 735

## Description

### TECHNICAL FIELD

The disclosure relates to an electrosurgical device. More specifically, it relates to an electrosurgical device for applying electrical energy to cut through tissue in a region of a patient's body.

### SUMMARY OF THE DISCLOSURE

The present invention is defined by the appended claims only, in particular by the scope of the appended independent claims. Reference(s) to "embodiments" throughout the description which are not under the scope of the appended claims merely represents possible exemplary executions and are therefore not part of the present invention. The following non-SI units can be converted into SI-units using the following conversion rule: 1 inch = 2.54 cm.

An electrosurgical device operable to traverse body vasculature to deliver a fluid and electrical energy about its distal end is disclosed herein. The electrosurgical device comprises: an electrically conductive elongate member for traversing body vasculature wherein electrical energy is deliverable, for example, through the wall of the elongate member; the elongate member defining a lumen; the elongate member further defining one or more distal apertures at or near its distal end, the one or more apertures being in fluid communication with the lumen; and an energy delivery device in electrical communication with the elongate member (i.e. with the wall defining the lumen) at or about the distal end of the elongate member. The energy delivery device includes an electrode for delivering energy. A thermal shield (also referred to herein as a spacer) is positioned between the electrode and the elongate member for thermally protecting portions of the electrosurgical device. The electrosurgical device is usable for traversing body vasculature of a patient and delivering a fluid and electrical energy from about its distal end.

In a first broad aspect, embodiments of the present invention comprise an electrosurgical device comprising: an elongate member including an electrically conductive wall for delivering electrical energy therethrough, the elongate member defining a lumen that is in fluid communication with at least one distal aperture; an energy delivery device including an electrode electrically coupled to the wall of the elongate member; and an electrically insulative thermal shield between the electrode and the elongate member. In some embodiments, the electrode is at the distal tip of the electrosurgical device.

As a feature of the first broad aspect, in some embodiments the electrosurgical device further comprises: a layer of insulation covering the elongate member; and the electrically insulative thermal shield being between the layer of insulation and the electrode for protecting the layer of insulation from heat generated by delivery of energy via the electrode. In some embodiments, a distal portion of the layer of insulation is distal to the elongate member distal end and covers or overlays a proximal portion of the thermal shield.

As another feature of the first broad aspect, certain embodiments include a flexible elongate member.

As another feature of the first broad aspect, in some embodiments of the electrosurgical device, the proximal end of the energy delivery device further comprises an energy delivery device coupler for electrically coupling with the elongate member. Some embodiments further comprise the energy delivery device having an intermediate portion for accommodating the thermal shield. Such embodiments can further include the energy delivery device coupler comprising an electrically conductive spacer fitting within and substantially blocking a distal part (including the distal end) of the lumen, with the electrically conductive spacer being in electrical communication with an electrically conductive surface of the wall of the elongate member. Furthermore, the energy delivery device can further comprise an intermediate conductive element extending between the electrically conductive spacer and the electrode, and, in some such embodiments, the thermal shield surrounds the intermediate conductive element. Typically, the electrically conductive spacer is cylindrical shaped and the intermediate conductive element is elongate. In some embodiments, the device further includes an electrode support (that may or may not form a part of the electrode; in some embodiments, the electrode support is integral with the electrode) that is disc shaped and supports a conductive dome electrode portion shaped like a segment of a sphere (for example, a hemisphere) formed on a surface of the electrode support. In some embodiments the thermal shield is generally cylindrical shaped and has a center bore for receiving the intermediate conductive element.

Certain embodiments of the first broad aspect include the elongate member comprising, for example, either a braided conductive layer, a metal layer with a helical configuration, or a metal tube with an interrupted helical groove cut into its outer surface. Other embodiments of the first broad aspect include the elongate member comprising non-metallic conductive materials.

In some embodiments of the electrosurgical device, the elongate member, electrode, and the electrically insulative thermal shield have outer diameters that range from about 0.014" to about 0.050". For some applications, embodiments of the device have dimensions (in particular outer diameter dimensions) that correspond with guide-wire sizes to facilitate the electrosurgical device being withdrawn and replaced with a guide-wire during a procedure i.e. the outer diameters of an electrosurgical device and guide-wire used in the procedure can be relatively close to facilitate exchange. Guide-wires used in applicable procedures are typically selected from the group consisting of guide-wires having outer dimension of about 0.014 inches, 0.018 inches, 0.025 inches, 0.035 inches, and 0.038 inches. As an example, to facilitate exchange with a 0.035 inch guide-wire, certain embodiments of the electrosurgical device include the elongate member having an outer diameter that ranges from about 0.033 to about 0.035 inches, the electrode having an outer diameter that ranges from about 0.032 to about 0.035 inches, and the electrically insulative thermal shield having an outer diameter that ranges from about 0.028 to about 0.031 inches. In a specific embodiment, the elongate member has an outer diameter of about 0.033 inches, the electrode has an outer diameter of about 0.032 inches, and the electrically insulative thermal shield has an outer diameter of about 0.028 inches. Embodiments can be dimensioned to facilitate exchange with other guide-wire sizes i.e. can have dimensions corresponding with the other guide-wire sizes.

The electrosurgical device can further comprise a support wire or spine member extending proximally from the electrically conductive spacer or some other part of the electrosurgical device, for adding stiffness and support to the elongate member, thereby facilitating advancement of the elongate member through body vessels. In general, there is no minimum spine length and the maximum support spine length is limited by the length of the lumen containing the spine. In some embodiments the support wire or spine can extend for a distance of about 3.835 inches (about 10 cm) or about 4 inches. In alternative embodiments, the support spine extends for a distance of at least about 3.835 inches (about 10 cm). In embodiments having a distal portion of the elongate member comprise a metal layer with cuts at least partially therethrough, the support spine can extend beyond the cut portion of the elongate member. In some embodiments, the support spine extends at least one centimeter proximal of the cut portion. Further details regarding the support spine are found in co-pending U.S. Provisional Patent Application Serial Number 61/777,368, filed 12-Mar-2013.

In some embodiments, elongate member 6 is between about 50 cm and about 120 cm in length, as is the lumen 26 defined therein. In such embodiments support spine can have a length between about 50 cm and about 120 cm in length. In embodiments of electrosurgical device 20 suitable for exchange, elongate member 6 is between about 50 cm and about 250 cm in length, as is the lumen 26 defined therein. In such embodiments support spine can have a length between about 50 cm and about 250 cm in length.

Some embodiments of the first broad aspect include the following features: the electrosurgical device (including the elongate member and the energy delivery device) having a fixed length; and the energy delivery device having a substantially atraumatic tip.

In some embodiments of the first broad aspect, the thermal shield has a thermal conductivity of at least 1 W/m-K, and/or the thermal shield is a ceramic. In alternate embodiments, the thermal shield has a thermal conductivity of at least 2 W/m-K. Some embodiments of the electrosurgical device include the thermal shield being comprised of a material having a thermal conductivity ranging from about 1 W/m-K to about 5 W/m-K, such as, for example, zirconium oxide and silicon carbide. Other embodiments of the electrosurgical device include the thermal shield being comprised of a material having a thermal conductivity ranging from about 15 W/m-K to about 40 W/m-K, such as, for example, zirconia toughened alumina (ZTA), sapphire crystal (aluminum oxide), and silicon nitride.

In a second broad aspect, embodiments of the disclosure are for a method to cross partial or total blockages, such as chronic total occlusions (CTOs), in vessels, for example peripheral vessels, that have tough (calcified) caps. In some embodiments of this broad aspect, the method comprises the steps of: (1) injecting a contrast fluid through an electrosurgical device positioned adjacent an occlusion; (2) assessing visibility of one or more channels through the occlusion using an imaging modality; (3) if no channels are visible in step 2, delivering energy from the electrosurgical device to the occlusion; and (4) repeating steps (1)-(3), as needed, until one or more channels through the occlusion are visualized. The method typically further comprises a step of delivering energy to the one or more visualized channels using the electrosurgical device for creating a pathway through the occlusion.

Some embodiments of the second broad aspect include a step (5) of delivering contrast fluid after cutting through the blockage to confirm the crossing.

In a third broad aspect, embodiments of the disclosure are for a method of cutting through an occlusion in a vessel of a patient, comprising the steps of: (i) positioning an electrosurgical device at a first desired location in the vessel substantially adjacent the occlusion; (ii) delivering energy using the electrosurgical device to at least partially cut through the occlusion; and (iii) measuring pressure using a pressure transmitting lumen defined by the electrosurgical device in order to determine the position of the electrosurgical device at least one of before and after step (ii). In some embodiments, step (i) comprises delivering contrast fluid through the electrosurgical device for confirming position at the first desired location. Some embodiments, further comprise the steps of: (iv) advancing the electrosurgical device to a second location; and (v) confirming position of the electrosurgical device at the second location using one or more of: a pressure measurement through a pressure transmitting lumen defined by the electrosurgical device; or delivering contrast fluid through the electrosurgical device.

Other methods of using the electrosurgical device comprise delivering cooling fluid and/or electrolytes through the lumen and aperture, and/or measuring pressure through the lumen and aperture.

### BRIEF DESCRIPTION OF THE DRAWINGS

In order that the invention may be readily understood, embodiments of the invention are illustrated by way of examples in the accompanying drawings, in which:
Fig. 1 is an illustration of an embodiment of an electrosurgical device of the present invention;
Fig. 2 is an illustration of detail A of Fig 1;
Fig. 3 is an illustration of detail B of Fig, 1;
Fig. 4 is an illustration of a spine support and attached distal region of the energy delivery device of Fig 1;
Fig. 5 is an illustration of detail A of Fig 4;
Fig. 6 is an illustration of an embodiment of an elongate member including a hypotube with cuts therein;
Fig. 7 is an illustration of an embodiment with an integral (single piece) energy delivery device;
Figs. 8a to 8d show an embodiment of the device that does not include a support spine;
Fig. 9 is an illustration of an embodiment having a step-down or tapered thermal shield;
Fig. 10 is a diagram illustrating the path of travel of a wire through an electrosurgical device;
Fig. 11 (in six sub-parts) is a diagram illustrating the steps of an embodiment of a method that includes delivering contrast fluid;
Fig. 12 is a flow chart illustrating an embodiment of a method that includes delivering cooling fluid;
Fig. 13 is a flow chart illustrating an embodiment of a method that includes delivering an electrolyte;
Figs. 14A and 14B illustrate embodiments having coiled markers;
Figs. 15A and 15B illustrate steps of an embodiment of a method used in a transseptal procedure; and
Figs. 16A and 16B illustrate examples with electrically conductive spines.

### DETAILED DESCRIPTION

Current devices used for CTO recanalization are generally solid flexible wires, such as guidewires. One of the greatest challenges in CTO (chronic total occlusion) recanalization is that such devices typically lack means to deliver fluid for visualization. The common current solution is to exchange the channeling device with a fluid delivery catheter. Other challenges to CTO recanalization exist specifically when using a device to deliver electrical or thermal energy to the CTO. For example, the energy delivered for channeling may damage parts of the device close to the heating zone.

The inventors of the instant application have conceived of, and reduced to practice, various embodiments of a device having features that address the above described challenges. The device includes a flexible elongate member comprised of an electrically conductive material. In addition, counterintuitively, the elongate member defines a lumen suitable for fluid delivery, while remaining suitably dimensioned for traversing vasculature and channeling through an occlusion. The device is configured for delivering energy to an electrode through the side-wall of the elongate member such that it is not necessary to include a conductive wire within the lumen to provide energy to the electrode. Utilizing a wall of the elongate member to conduct the electrical energy, thereby leaving the fluid delivery portion of the lumen substantially free of obstruction, enables the device to have a diameter suitable for delivery to, and channeling through, a CTO. Embodiments of the disclosed device further include a heat-shield between the electrode and the elongate member to protect the elongate member from being damaged by heat.

In embodiments including an electrically insulative heat/thermal shield, the present inventors have further conceived and reduced to practice a means of maintaining a conductive pathway between the wall of the elongate member and the electrode. For example, the disclosed device also includes structure(s) defining the electrical pathway, where the structure(s) is/are small and structurally sound. The electrode and the structure(s) providing the pathway to the elongate member together form an energy delivery device. In some embodiments of the device, an electrically conductive support spine extends from the energy delivery device proximally through a portion of the lumen to provide a secondary electrical path from the elongate member to the electrode while simultaneously providing structural support to the elongate member, particularly during bending or curving of the device.

In addition, the present inventors have conceived of and reduced to practice novel methods of medical treatment, which are not claimed.

Some of the disclosed methods include channeling through chronic total occlusions within vasculature and delivering fluids using a single medical device rather that separate devices for energy delivery and fluid delivery.

With specific reference now to the drawings in detail, it is stressed that the particulars shown are by way of example and for purposes of illustrative discussion of certain embodiments of the present invention only. Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways, limited by the scope of the claims. . Also, it is to be understood
that the phraseology and terminology employed herein is for the purpose of description and should not be regarded as limiting.

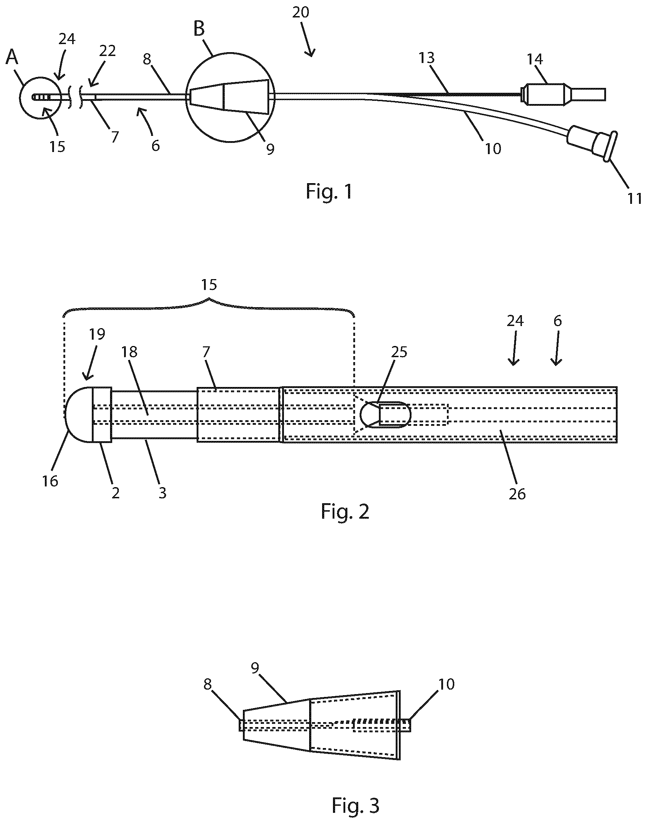

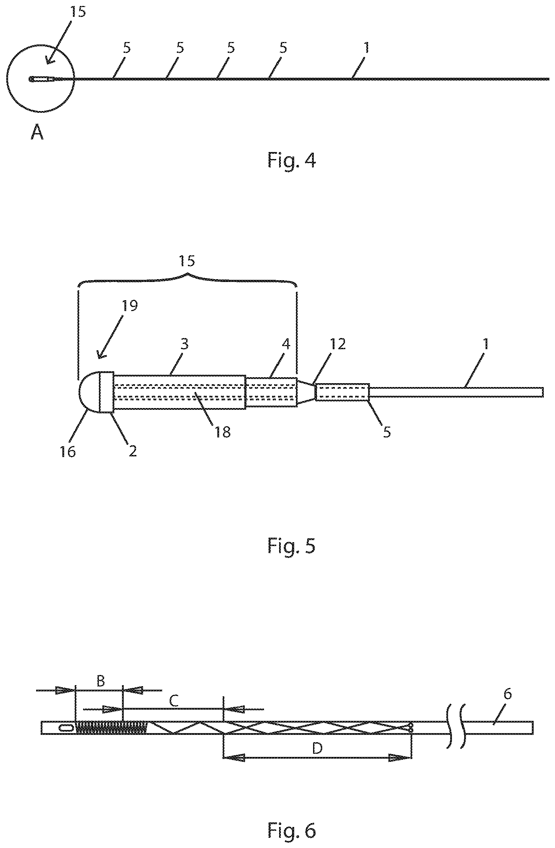

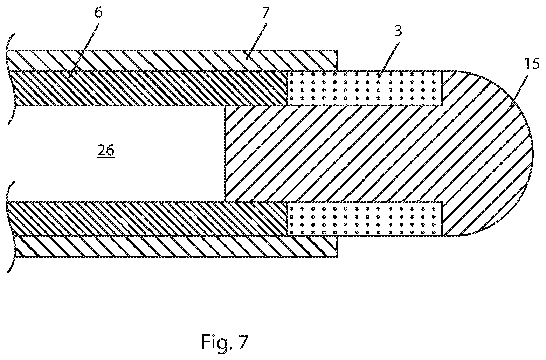

Fig. 1 illustrates an embodiment of a medical device 20, which comprises an elongate member 6 having a proximal region 22 and a distal region 24, and an energy delivery device 15 (Fig. 2 and, in greater detail, Fig. 5) associated with the distal end of device 20. Elongate member 6 is tubular in configuration defining at least one lumen 26 (Fig. 2) extending substantially throughout its length, and is electrically conductive for conducting energy along the length of elongate member 6 to energy delivery device 15. In some embodiments, elongate member 6 is a hypotube. In addition, in typical embodiments, elongate member 6 defines at least one aperture 25 in a wall thereof (Fig. 2), which in typical embodiments is a distal aperture, i.e. an aperture which is at or near the distal end of elongate member 6 or medical device 20. The distal aperture and the lumen defined by the elongate member combine to form a pressure transmitting lumen, whereby fluid pressure from an external environment on the aperture is transmitted through a column of fluid located in the lumen to be measured at a proximal portion of the device. For example, the medical device may be operable to be coupled to a pressure sensing mechanism, such as a pressure sensor, to measure the pressure transmitted through the lumen. Medical device 20 further comprises a hub 9 (shown in detail in Fig. 3) associated with the proximal region 22 of elongate member 6. While the embodiment of elongate member 6 of Fig. 1 is biased towards a straight configuration, as illustrated, elongate member 6 is flexible enough to bend when being advanced through a curved lumen. Some alternative embodiments of elongate member 6 include a curved portion (e.g. Fig. 7).

With reference now to Fig. 2, fluid can be delivered through aperture 25 (which also extends through insulation layer 7). For example, a physician using electrode 19 to cross (or channel through) an occlusion in a body vessel could inject a contrast fluid, for example, through lumen 26 and one or more apertures 25 (or sideports 25) and use x-ray fluoroscopy or another imaging modality to confirm how far the device has progressed in cutting or channeling through the occlusion. The apertures typically are large enough to allow fluid to flow through them.

Fig. 2 illustrates an embodiment of electrosurgical device 20 comprising insulation layer 7 disposed on top of, over, or around the distal region 24 of elongate member 6. Insulation layer 7 extends substantially from proximal region 22 to distal region 24 of elongate member 6. Insulation layer 7 may be made from an electrically insulative material such as PEBAX^{®} (polyether block amide), PEEK (Polyether ether ketone), PTFE (Polytetrafluoroethylene), or another thermoplastic or polymeric material. In some embodiments, insulation layer 7 is at least partially pervious to light, for example insulation layer 7 is fabricated from a transparent Nylon. Insulation layer 7 may be applied to elongate member 6 by a variety of means. In one embodiment, insulation layer 7 is extruded over elongate member 6. In another embodiment, insulation layer 7 is manufactured as a pre-formed cylinder, which is placed over elongate member 6, and subjected to heat to tighten (i.e. recover) insulation layer 7 around elongate member 6. In another embodiment, insulation layer 7 is applied to elongate member 6 by dip-coating or spraying. As the outer diameter of elongate member 6 ranges from about 0.010 to about 0.050 inches, the inner and outer diameters of insulation layer 7 vary accordingly. In one embodiment of electrosurgical device 20 is exchanged with a 0.035 inch guide-wire, insulation layer 7 has a recovered inner diameter of about 0.028 to 0.030 inches and a recovered outer diameter of about 0.034±0.001 inches (after heat shrinking).

There is electrical continuity between distal region 24 of elongate member 6 and electrode 19 of energy delivery device 15 whereby electrical energy being conducted along the elongate member reaches electrode 19. As insulation layer 7 is present over substantially the entire elongate member (which may be metallic, e.g. a hypotube), the path of least resistance for electrical energy flowing through elongate member 6 is through the electrode 19.

A thermal shield 3, located proximal of electrode 19, protects the integrity of the rest of device 20. Thermal shield 3 is an electrical and thermal insulator that functions to insulate and thus protect the distal region 24 of the elongate member (e.g. insulation layer 7), from the heat generated at electrode 19 and functions to prevent arcing between the electrode and the elongate member. Typical embodiments of electrosurgical device 20, such as the example shown in Fig. 2, include insulation layer 7 extending over (or overlapping) a proximal portion of thermal shield 3. Alternative embodiments do not include such an overlap.

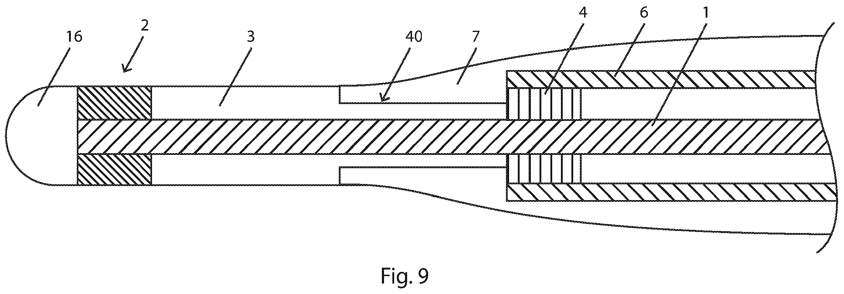

In embodiments having the above- described overlap of insulation layer 7, the overlap of the layer of insulation over the thermal shield 3 forms a sealed junction. This may help minimize arcing observed behind (i.e. proximal of) the thermal shield 3 near the junction and may help minimize degradation of insulation layer 7 from the heat generated at electrode 19 (or in surrounding tissue) from the delivery of electrical energy through electrode 19. The overlap of insulation layer 7 with thermal shield 3 can also serve to prevent fluid from leaking from the distal portion of lumen 26 of electrosurgical device 20. In some embodiments, thermal shield 3 can have a taper or step 40, as shown in Fig. 9, to provide a smoother outer diameter transition between insulation layer 7 and thermal shield 3. In some such embodiments, the taper or step 40 is substantially gradual while in other embodiments the change in diameter is relatively discrete/abrupt.

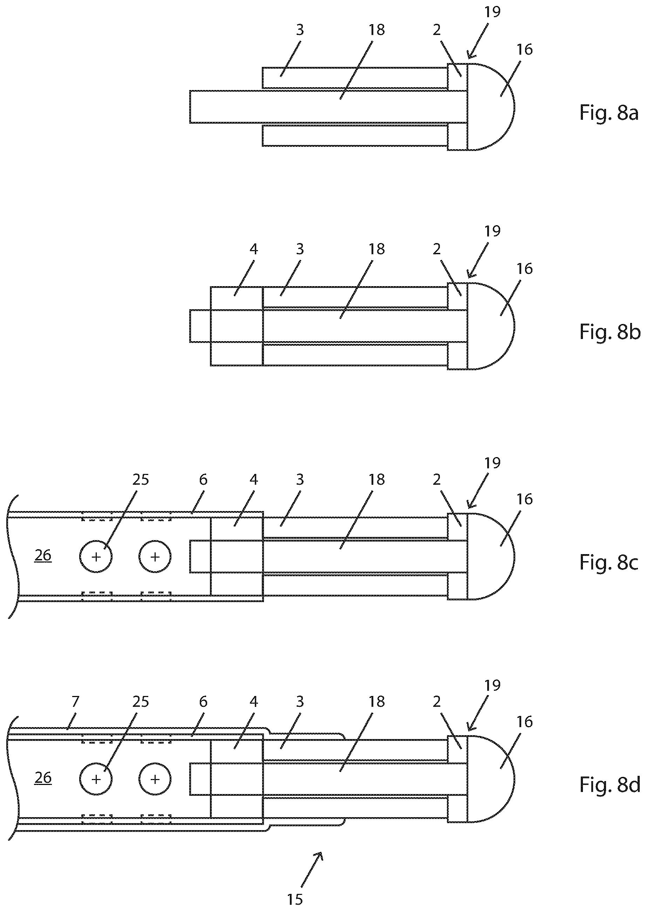

In some embodiments, elongate member 6 defines one or more apertures 25, for example as shown in Figs. 2 and 8d. Aperture(s) 25 allow for fluid communication between the outside environment and lumen 26.

In the embodiment of Fig. 1, proximal region 22 is coupled to a hub 9 (shown in detail in Fig. 3), which is coupled to flexible tubing 10 for allowing the electrosurgical device to be in fluid communication with fluid connector 11. Flexible tubing 10 can comprise a polymeric material, for example, Tygon^{®} tubing, polyvinylchloride (PVC), or another flexible polymer. Fluid connector 11 can be, for example, a Luer lock and is structured to be operatively connected to a source of fluid, for example a syringe or an aspirating device, or to a pressure sensing device, for example a pressure transducer.

Electrosurgical device 20 further includes means for electrically coupling proximal region 22 of elongate member 6 to an energy source. In the embodiment illustrated in Fig. 3, proximal region 22 connects to hub 9 and insulated wire 13 (shown in Fig. 1) is electrically coupled to proximal region 22 within hub 9. The proximal end of insulated wire 13 is connected to electrical connector 14 (or plug 14), which is be electrically coupled to a source of energy, for example a generator. Strain relief 8 (Figs. 1 and 3) provides for a transition of stiffness between proximal region 22 of elongate member 6 and hub 9.

Hub 9 also functions as a handle for a physician when electrosurgical device 20 is in use. In some embodiments, the hub (or handle) can be removed to allow proximal end loading of devices onto or into elongate member 6 for advancement thereupon or therethrough.

Some examples of electrosurgical device 20 include a support spine 1 (also referred to as a support wire/stiffening member), for example as shown in Figs. 4 and 5. Spine 1 functions to provide additional stiffness to electrosurgical device 20 while leaving lumen 26 substantially open or unobstructed for flow of fluid, such as imaging or contrast fluid. In some embodiments of electrosurgical device 20, the elongate member comprises a metal layer with a flexible helical configuration and wherein the support spine provides support to the wall of the elongate member. In some embodiments, spine 1 is comprised of nitinol and can provide shape memory properties to the electrosurgical device. In some embodiments incorporating a spine 1, the spine is an electrical conductor that extends proximally from energy delivery device 15 to make an electrical connection with a conductive wall of lumen 26. In some such embodiments, spine 1 extends proximally beyond conductive spacer 4. In some embodiments, the support spine extends proximally beyond conductive spacer 4 for a distance of at least about 10 cm. In some such embodiments, the support spine extends proximally for a distance in a range of between about 10 cm to about 120 cm. In other embodiments, support spine extends proximally for a distance in a range of between about 10 cm to about 250 cm. In alternative embodiments, spine 1 is electrically non-conductive.

In the example illustrated in Fig. 5, spine 1 includes flare 12 (a barb). In some examples, flare 12 is separate from and coupled to spine 1 while in alternative examples flare 12 is integral with spine 1. Flare 12 can retain conductive spacer 4 in place. In alternative examples that lack flare 12, a band marker 5 (further details below) could be positioned adjacent conductive spacer 4 to retain the spacer in place.

Further details regarding the support spine are found in co-pending U.S. Provisional Patent Application Serial Number 61/777,368, filed 12-Mar-2013.

Some embodiments, for example as illustrated in Fig. 5, include an intermediate conductive element 18 for conductive electrical energy between components of the electrosurgical device, for example between electrode 19 and spine 1. In some such examples, intermediate conductive element 18 is an extension of spine 1. In alternative examples, intermediate conductive element 18 is a separate part distinct from spine 1, such as, for example, a wire or rod.

In some embodiments, one or more visualization markers, such as radiopaque markers 5 (e.g. Figs. 4, 5), is associated with electrosurgical device 20 to highlight the location of important landmarks on electrosurgical device 20 using a medical imaging modality. Such landmarks include the location of energy delivery device 15 or the location of any aperture(s) 25. Typically, radiopaque markers provide the radiopacity to more readily visualize the device under fluoroscopy. Radiopaque marker 5 can be comprised of platinum or any other suitable radiopaque material. In alternative embodiments, other forms of markers are employed for visualization using alternate imaging modalities - for example, echogenic markers are utilized to enable visualization using ultrasonic imaging.

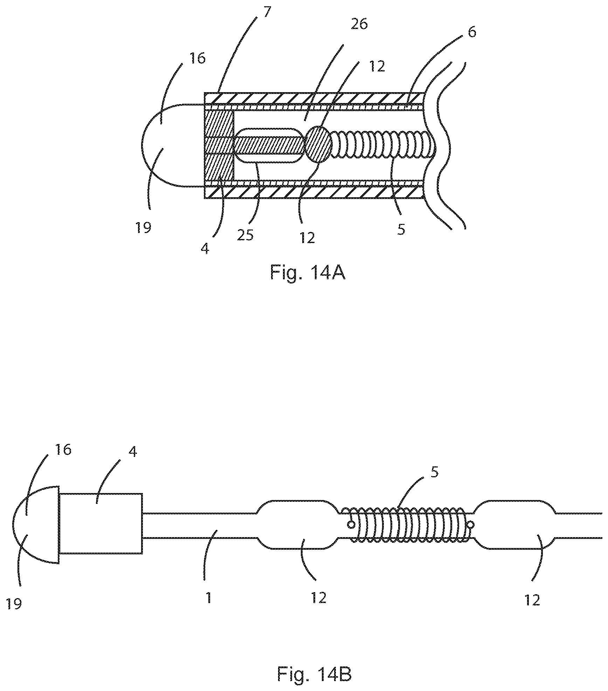

Some alternative embodiments have a spiral or a coiled marker 5 rather than band markers. In the examples of Figs. 14A and 14B, coiled markers 5 are installed on spine 1. Each flare (or protrusion) 12 acts as a restraint to prevent coiled marker 5 from travelling along spine 1. In the example of Fig. 14B, each flare 12 is comprised of flattened spine 1 (or flattened wire). In other alternative embodiments, one or both ends of a coiled marker could be fixed in place by laser welding or crimping. A coiled marker 5 is typically comprised of platinum or tungsten. In the embodiment of Fig. 14A, the coiled marker is proximal of the aperture 25 whereby it can assist in positioning the aperture within a patient's body. For example, by positioning coiled marker 5 outside (distal) of a dilator, a physician would ensure that aperture 25 is also outside of the dilator and could be used to deliver or aspirate fluids and/or measure pressure.

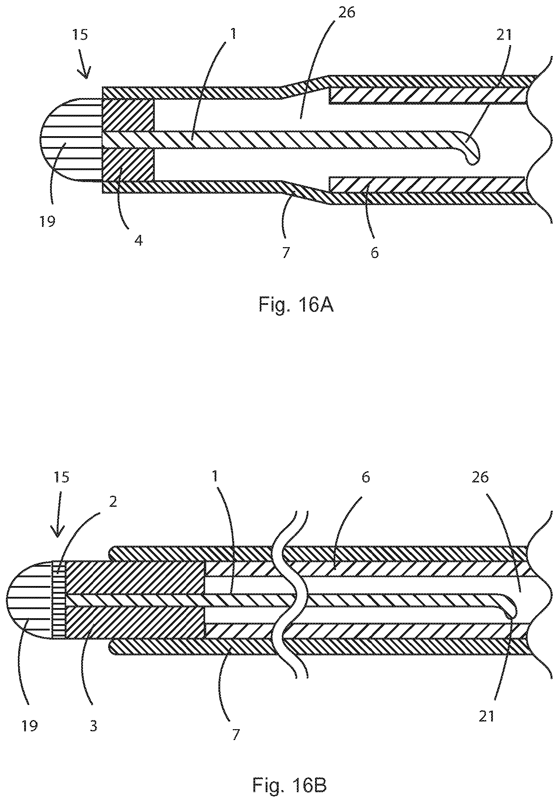

Energy delivery device 15, of the embodiment of Fig. 5, is comprised of: an electrode 19, an intermediate conductive element 18 that is attached to and in electrical communication with electrode 19, and a conductive spacer 4 that is attached to and in electrical communication with intermediate conductive element 18. While different configurations of electrode 19 are possible, Fig. 5 discloses an embodiment including support structure 2 and a conductive dome 16. In addition, while Fig. 5 illustrates an energy delivery device associated with a spine 1, other embodiments incorporate an energy delivery device 15 (including, for example, electrode 19) without having a spine 1. Support structure 2 can be metallic, puck or disk-shaped and, in some embodiments, is comprised of tantalum. Conductive spacer 4 may be comprised of, for example, nitinol, gold, stainless steel or platinum. Conductive dome 16 may be formed by laser welding a metal, possibly the end of intermediate conductive element 18, onto support structure 2. In alternative embodiments, conductive spacer 4 is replaced by other energy delivery device coupler elements for attachment of energy delivery device 15 to elongate member 6. In some such examples, the energy delivery device coupler comprises an electrically conductive support spine (e.g. Fig. 16).

Electrode 19 is configured and sized such that a sufficiently high current density may be provided at electrode 19 to generate arcing in a region of tissue when electrode 19 is positioned proximate the region of tissue. This allows a channel to be created through at least a portion of the region of tissue. For details regarding electrosurgical arcing and channel creation, reference is made to U.S. patent application serial number 12/926,292.

As illustrated in Fig. 5, thermal shield 3 is mounted about intermediate conductive element 18. In some embodiments, thermal shield 3 is a ceramic, for example sapphire, zirconia or alumina. Intermediate conductive element 18 is comprised of any suitable conductive material that is electrically coupled to the other components of electrosurgical device 20 as described herein.

Electrode 19 is electrically and operatively coupled to energy delivery device 15 by a variety of means, including gluing or insert molding, for example. The electrode is made from any suitable electrically conductive material. Examples of suitable materials include stainless steels, copper, and platinum.

The electrode may be one of various shapes and sizes, for example, substantially cylindrical, and/or having a hemispherical, rounded, or domed end.

Making reference to Figs. 2 and 5, energy (such as electrical energy) is operable to be delivered through the wall of elongate member 6 rather than through a separate conductive element (e.g. a wire) located within lumen 26 (other than spine 1, which typically does not extend proximally throughout lumen 26). This allows sufficient space in lumen 26 for the flow of a fluid that can exit through (or be drawn from) apertures 25. In some such embodiments, electrode 19 is electrically coupled to an inner wall of elongate member 6 while the insulation layer 7 is thermally insulated from electrode 19 by means previously described (e.g. using thermal shield 3). In the embodiments illustrated in, for example, Figs. 2 and 5, a single electrosurgical device 20 having, for example, outer diameters that range from about 0.014" to about 0.050", can adequately deliver both energy (for example, along the wall of elongate member 6 to electrode 19) and fluid (through lumen 26 and aperture(s) 25).

In some embodiments, where elongate member 6 is fabricated from a super-elastic material, the advancement of electrosurgical device 20 through tortuous vasculature is facilitated by the superelasticity of elongate member 6.

Elongate member 6 is electrically coupled to energy delivery device 15 by a variety of connecting means. For example, in one embodiment, elongate member 6 can be welded or soldered to conductive spacer 4 of energy delivery device 15.

Embodiments of elongate member 6 are made from a number of different materials. Examples include stainless steel, copper, nickel, titanium, and alloys thereof. Some embodiments include elongate member 6 being a stainless steel hypotube or a nitinol hypotube.

In some embodiments, notches are cut into elongate member 6, by, for example, laser-cutting, such that the region comprising the notches is relatively more flexible (than it would be without such cuts) while retaining sufficient conductivity along elongate member 6. Different configurations of cuts are possible, including: c-cuts, spiral shaped cuts, interrupted spiral cuts, interlocking cuts and dove-tail cuts. Furthermore, the cuts or notches may be made partially or completely through a wall of elongate member 6.

The specific embodiment of Fig. 6 discloses cuts into elongate member 6 that include constant pitch portion B, variable pitch portion C and dual pitch potion D. Elongate member 6 of Fig. 6 may be a hypotube. The cuts into the hypotube may traverse the hypotube wall. Having a smaller pitch (i.e. the cuts closer together) increases the flexibility of elongate member 6. Considering the example of variable pitch portion C, the part of variable pitch portion C closer to the distal end of elongate member 6 has a smaller pitch than the part of portion C that is further from the distal end of elongate member 6 and consequently is be more flexible. Alternatively, the flexibility of elongate member 6 is increase by incorporating a dual pitch (i.e. two cut lines, which may intersect with one another) instead of a single pitch, such as in the example of dual pitch portion D.

This variable flexibility assists in the proper positioning and use of electrosurgical device 20 in surgical procedures. For example, a more flexible distal region 24 is desirable for navigating through conduits, for example blood vessels, in a patient's body, while a stiffer proximal region 22 is desirable to allow for pushability, or resistance to kinking under axial compression force, of the device. A relatively stiffer proximal region 22 is desirable for torque response and radial rigidity. It is also possible that, for example, two different embodiments of elongate member 6 could have different wall thickness dimensions and/or different outer diameters (with each embodiment having constant thickness and diameter dimension along its length) to vary flexibility. It is further possible that a single embodiment of elongate member 6 could have different wall thickness dimensions and/or different outer diameters along its length to vary flexibility.

In some embodiments of electrosurgical device 20 incorporating a spine 1 as described hereinabove, in which elongate member 6 is normally biased to be straight, the shape memory properties and stiffness of spine 1 allow electrosurgical device 20 to display the stiffness and response of a guide-wire, to revert to a straight configuration after being bent and to compensate for flexibility created by cuts into elongate member 6. In addition, spine 1 can act as a bridge across the cuts to distribute the bending stress along elongate member 6, for example as described with reference to Figs. 25A and 25B of U.S. Provisional Patent Application Serial Number 61/777,368.

In some embodiments, elongate member 6 is between about 50 cm and about 120 cm in length, and has an inner diameter of about 0.028 inches and an outer diameter of about 0.032 inches. Embodiments of this length have a fixed (not removable) hub (or handle). Other embodiments have an inner diameter of about 0.025 inches and an outer diameter of about 0.029 inches. The dimensions of elongate member 6 depend on one or more factors, including: the distance to the target site, the tortuosity and/or diameter of the vessel(s) to be navigated, whether or not the elongate member is desired to be exchange length (e.g. about 50 cm to about 2.5 m), and any other requirement imposed by auxiliary devices to be used with elongate member 6. For example, elongate member 6 is typically sized such that it is compatible with a sheath and/or dilator within which it is to be disposed.

Alternative configurations of the energy delivery device 15 are possible. The embodiment of Fig. 7 illustrates an example of an integral (single piece) energy delivery device 15. Conductive spacer 4, intermediate conductive element 18 and electrode 19 comprise a single element or part, i.e. they are all incorporated into a single, unitary energy delivery device 15. In some such embodiments, thermal shield 3 is attached to energy delivery device 15 by gluing or pressure fit, and/or is retained in place, at least in part, by insulation layer 7.

### Embodiment without support spine (Figs. 8a-d)

Figs. 8a to 8d disclose an embodiment of the electrosurgical device 20 that comprises an intermediate conductive element 18 that comprises a metal rod. The electrosurgical device is operable such that electrical energy flows through the wall of elongate member 6, conductive spacer 4, and intermediate conductive element 18 to tip electrode 19, as indicated by the arrows in Fig. 8d showing the flow of electrical energy along the device. The embodiment of Figs. 8a-d lacks an extended support spine, such as shown in the embodiments of Figs. 4-5, thereby further facilitating fluid flow through lumen 26. As described above, this embodiment allows for delivery of electrical energy to electrode 19, via the wall of elongate member 6, conductive spacer 4 and intermediate conductive element 18, as well as the delivery and/or aspiration of fluid through lumen 26 and aperture(s) 25. This particular embodiment includes eight circular apertures (six are shown in the figures, with another two in the wall that is cutaway in the figures) but the number and shape of aperture(s) 25 can vary.

In some embodiments of medical device 20, the support spine 1 functions as the primary (or only) pathway for electrical energy to travel from elongate member 6 to energy delivery device 15. In the example of Fig. 16A, conductive spacer 4 is spaced apart from and not in contact with elongate member 6. Insulation layer 7 is a continuous layer of one material in Fig. 16A, but in alternative embodiments insulation layer 7 could be comprised of more than one type of material: for example, the portion of insulation layer 7 covering elongate member could be one (or more) type of material and the portion of insulation layer 7 distal of elongate member 6 could a different material (or materials). In general, at least some portion of insulation layer 7 distal of elongate member 6 is comprised of electrically non-conductive material, whereby electrical energy cannot flow through insulation layer 7 to energy delivery device 15. The embodiment of Fig. 16A includes a spinal curve 21 to facilitate support spine 1 contacting elongate member 6. Some alternative embodiments have a bend. Other alternative embodiments of medical device 20 could have a generally straight support spine 1 (i.e. lacking spinal curve 21) or some other configuration, for example, a helical spring. Whether the distal portion of support spine 1 is curved, bent, straight, or some other configuration, support spine 1 is typically sufficiently elongate and floppy to facilitate it contacting elongate member 6 at some position along its length. While conductive spacer 4 is typically a metallic material to facilitate welding electrode 19 to medical device 20 and securing support spine 1, in alternative embodiments, conductive spacer 4 could be a non-metallic material and/or an electrically non-conductive material.

Making reference to Fig. 16B, some examples of medical device 20 have a thermal shield 3 comprised of non-conductive material whereby support spine 1 functions as the primary pathway for electrical energy to travel from elongate member 6 to energy delivery device 15. While thermal shield 3 is a single integral part in some embodiments (e.g. Fig. 16B), in alternative embodiments, thermal shield 3 could be comprised of more than one part and/or material. The embodiment of Fig. 16B does not include a conductive spacer 4 proximal of thermal shield 3 (as seen in the example of Fig. 5) to enable electrical communication between elongate member 6 and electrode 19. Some embodiments include an energy delivery device 15 comprised of support structure 2 and electrode 19, such as the example of Fig. 16B; alternative embodiments have other energy delivery device configurations, e.g. the distinct support structure 2 and electrode 19 being replaced by a single integral part. While the embodiment of Fig. 16B includes a spinal curve 21, alternative examples of medical device 20 have a distal portion of support spine 1 that is bent, straight, or some other configuration. Alternative examples include a flare 12, as previously described in reference to Fig. 5. In both of the examples illustrated in Figs. 16A and 16B, a non-conductive material restricts or impedes the electrical pathway from elongate member 6 to electrode 19 such that support spine 1 is the primary (or only) pathway of electrical conductivity between elongate member 6 and electrode 19. The non-conductive material could be, for example, a ceramic or a polymer.

Sources of energy that may be used in conjunction with electrosurgical device 20 include, for example, generators of ultrasonic, microwave, radiofrequency or other form of electromagnetic energy. In embodiments utilizing ultrasonic energy, energy delivery device 15 may comprise an ultrasound transducer. In one particular embodiment, the source of energy is a radiofrequency (RF) electrical generator, operable in the range of, for example, about 100 kHz to about 3000 kHz, designed to generate a high voltage in a short period of time. More specifically, the voltage generated by the generator may increase from about 0 Vrms to greater than about 400 Vrms in less than about 0.6 seconds. The maximum voltage generated by the generator may be between about 180 V peak-to-peak and about 3000 V peak-to-peak. The waveform generated may vary, and may include, for example, a sine-wave or a rectangular wave, amongst others. In some embodiments, due to the small size of the electrode, the impedance encountered during RF energy application may be very high and the generator is operable to deliver energy notwithstanding the increased impedance. The generator may be operable to continue to maintain the desired voltage when the impedance of the tissue changes or is low. Output impedance of a suitable generator may be between 100 ohm and 200 ohm. In one particular example, energy is delivered to a tissue within a body at a voltage that rapidly increases from 0Vrms to 400 Vrms. Alternate embodiments of suitable radiofrequency generators have power capabilities of 0 to 25 watts, 0 to 50 watts, or 0 to 300 watts.

In one broad aspect, electrosurgical device 20 is used to deliver energy to a target site within a body of a human or animal while concurrently or sequentially delivering a fluid via aperture(s) 25.

In some embodiments, the energy may be radiofrequency (RF) current, and the energy may function to puncture or create a void or channel in the tissue at the target site. RF energy is delivered in such a way it may result in the creation of an insulative vapor layer around the electrode, therein resulting in an increase in impedance, for example the impedance may increase to greater than 4000Ω. Increasing the voltage increases the intensity of fulguration, which may be desirable as it allows for an increased tissue puncture rate. An example of an appropriate generator for this application is the BMC RF Puncture Generator (Model numbers RFP-100 and RFP-100A, Baylis Medical Company, Montreal, Canada). These generators can deliver continuous RF energy at about 480 kHz. A grounding pad or dispersive electrode is connected to the generator for contacting or attaching to a patient's body to provide a return path for the RF energy when the generator is operated in a monopolar mode.

Further details regarding delivery of energy to a body may be found in U.S. Patent Applications 10/347,366 (filed on January 21st, 2003), 10/760,749 (filed on January 21st, 2004), 10/666,288 (filed on September 19th, 2003), and 11/265,304 (filed on November 3rd, 2005), and U.S. Patent 7,048,733 (Application 10/666,301, filed on September 19th, 2003).

In some embodiments, electrosurgical device 20 may be used to create a channel through an occluded lumen or other material within the body. Examples may include blood vessels, stent-graft fenestrations, the bile duct, or airways of the respiratory tract. An occlusion may comprise fibrous tissue or other material, and the occlusion may be partial or substantially complete. In some embodiments, electrosurgical device 20 is positioned such that the electrode is adjacent the material to be punctured. Energy may be delivered from a source, such as a generator, via elongate member 6, to the target site such that a void, or channel, is created in or through the tissue. Further details regarding delivery of energy to create channels through tissue or occlusions may be found in U.S. Patent Application 12/926,292, filed on November 8, 2010, U.S. Patent Application 13/286,041, filed on October 31, 2011, and U.S. Patent 8,048,071, issued November 1, 2011.

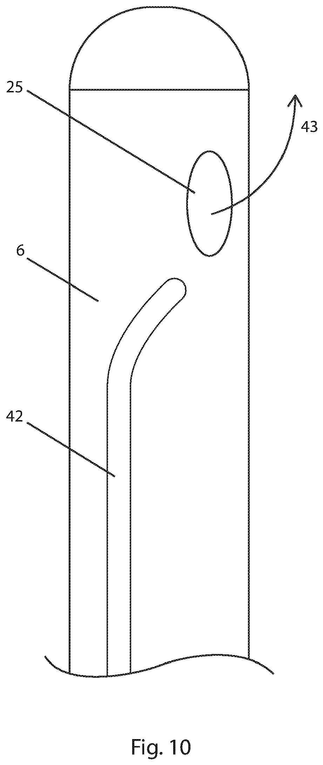

When widening or dilating occlusions after crossing with electrosurgical device 20, device 20 can be replaced with a guide-wire prior to installation or insertion of a device used for widening, such as a dilator or balloon catheter. In one method of using electrosurgical device 20, a guide-wire can be installed before removing device 20 by advancing a wire 42, for example with a curved or bent distal end, through lumen 26 and out of an aperture 25, such as indicated by arrow 43 in the diagram of Fig. 10. The wire 42 can be advanced under imaging and when the distal end of wire 42 has advanced sufficiently to reach aperture 25, wire 42 may be rotated, if required, to locate aperture 25. Electrosurgical device 20 can then be removed from the body and a dilation device may be inserted over wire 42. Alternatively, widening devices can be loaded onto or over electrosurgical device 20, in front of (i.e. distal to) hub 9, prior to inserting device 20 into the vasculature and/or prior to utilizing device 20 is used to cross the occlusion. As previously described, it is also possible to use an electrosurgical device with a removable hub 9 to facilitate proximal-end loading of devices onto electrosurgical device 20.

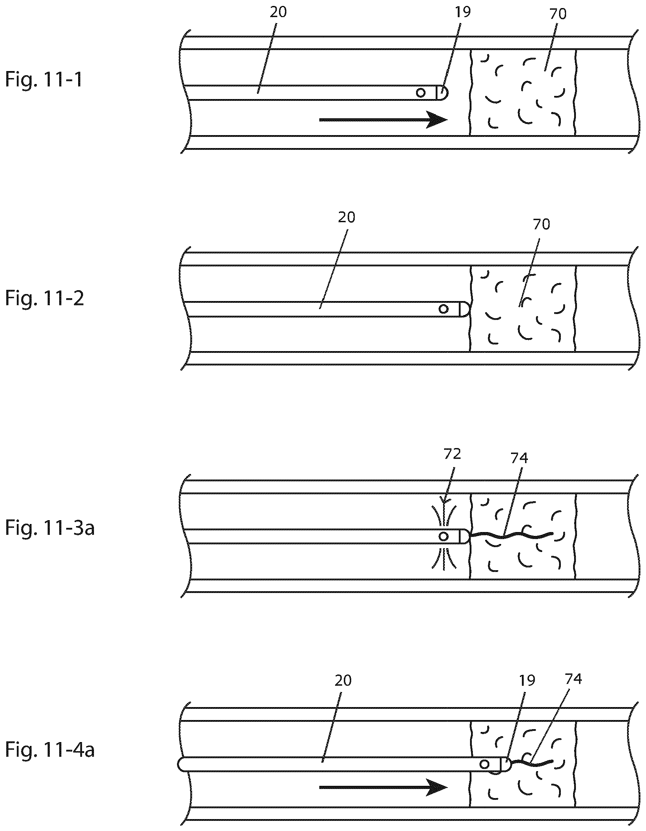

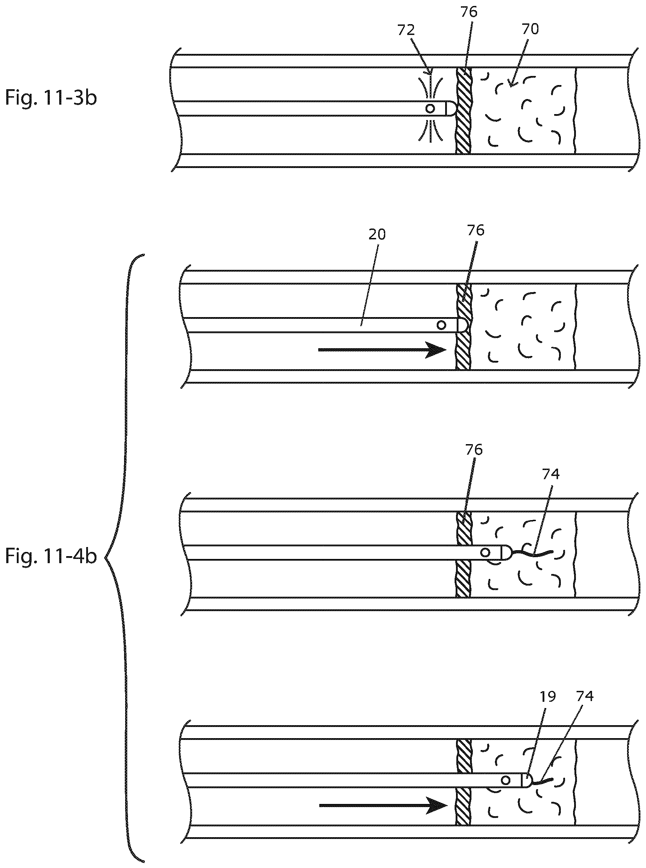

In some embodiments, an exemplary unclaimed method includes delivering contrast or imaging fluid to the treatment site to provide information about the environment to the physician using a medical imaging modality. For example, contrast fluids can be injected into CTOs (chronic total occlusions) in the coronary vessels to expose microvessels (pathways) within the occlusion under fluoroscopy, allowing the pathways to be visualized. Some CTOs have tough proximal caps that are difficult to cross. Referring to Fig. 11, an embodiment of a method of crossing CTOs in peripheral vessels that may have tough (calcified) caps comprises:
1) inserting electrosurgical device 20 into the vessel and advancing to the CTO 70 site in the vessel (Fig. 11 step 1);
2) engaging the CTO 70 (Fig. 11 step 2);
3) injecting contrast fluid 72 and checking for channels 74 through the occlusion using fluoroscopic imaging (Fig. 11 step 3a or 3b);
4a) if pathways or channels 74 are seen, using them as a guide to cut through the CTO by delivering energy through electrode 19 (Fig. 11 step 4a); and
4b) if no pathways are seen in step (3), delivering energy through electrode 19 to cut through the proximal cap 76 (which may be preventing contrast fluid from accessing these microchannels), injecting additional contrast fluid (e.g. once the proximal cap is traversed) and, if pathways 72 are visible, using any such visible pathway 72 as a guide to cut through the remainder of the CTO 70 by delivering energy through electrode 19 (Fig. 11 step 4b). If no pathways are visible, energy continues to be delivered as the device advances through the occlusion. These steps may be repeated in order to check for pathways through the occlusion as the device is being advanced.

After the steps described in the above method are completed and the CTO is crossed, more contrast fluid can be delivered to confirm the crossing.

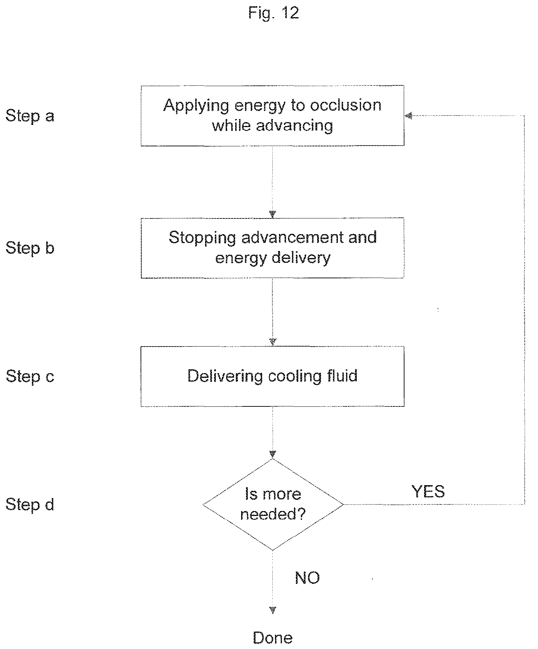

Some methods of using electrosurgical device 20 to cross CTOs or other occlusions or stenoses include delivering fluid for cooling the tissue around the occlusion to avoid damaging the tissue by the delivery of energy. One embodiment (as shown in Fig 12) comprises the steps of: a) applying RF energy to the occlusion through electrode 19 while advancing electrosurgical device 20, b) stopping advancement of electrosurgical device 20 and the application of energy c) delivering about 1 cc to about 2 cc of cooling liquid to the treatment site, and d) repeating steps (a) through (c) as needed. In some embodiments, the generator used to supply the energy in step (a) can be set at power levels ranging from about 0 to about 70W. In some embodiments of the method, energy is delivered for a time period of from about 50 microseconds to about 5 seconds. Some embodiments include multiple, or pulsed, deliveries of energy. Energy is delivered at power levels and time periods appropriate for the occlusion and surrounding tissue. The fluid delivered for cooling in step (c) may include bio-compatible saline. Some embodiments of a method aspect of the present disclosure include delivering contrast fluid for imaging during step (c). In the embodiments of Fig. 12, energy and fluid delivery are sequential.

Other methods of using electrosurgical device 20 to cross CTOs include delivering energy on a substantially continuous and constant basis, while advancing and delivering cooling fluid at least partly concurrently with the delivery of energy. In such embodiments, energy delivery is maintained while fluid delivery may be stopped or started during the procedure. Thus, at certain points in the procedure energy and fluid are delivered simultaneously, while at other points only energy is being delivered.

Further methods of using electrosurgical device 20 to cross occlusions such as CTOs include continuously delivering fluid while advancing the device and delivering energy as needed while delivering fluid. The energy can be delivered continuously or intermittently. In such embodiments, delivery of fluid is maintained, while the delivery of energy may be terminated or initiated during the procedure. As in the previous embodiment, at certain points in the procedure energy and fluid are delivered simultaneously, while at other points only fluid is being delivered. Discontinuous delivery may include delivering energy or fluid in short pulses, repeating longer periods of delivery, or intermittent delivery controlled by the physician.

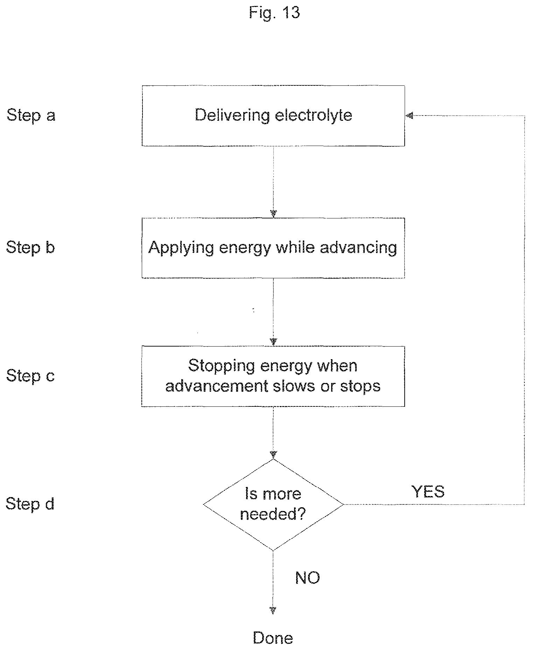

Injected fluid (e.g. saline) may also serve as an electrolyte to improve the efficacy of the energy being delivered to the tissue. One embodiment of a method of using electrosurgical device 20 to cross occlusions such as CTOs that includes delivering electrolyte fluid to the treatment site (Fig. 13) to facilitate cutting comprises the steps of: a) delivering electrolyte liquid to the treatment site for a controlled amount of time (and terminating delivery of fluid), b) applying RF energy to the occlusion through electrode 19 while advancing electrosurgical device 20, c) stopping the delivery of energy when advancement of electrosurgical device 20 has slowed or stopped (i.e. has been impeded) and d) repeating steps (a) through (c) as needed to advance through an occlusion.

Any of the above described methods of crossing CTOs may include using a slow continual flow of contrast fluid from the aperture as electrosurgical device 20 is advanced such that a small trail of contrast fluid is left in the channel thereby creating visible trace of the pathway of electrosurgical device 20.

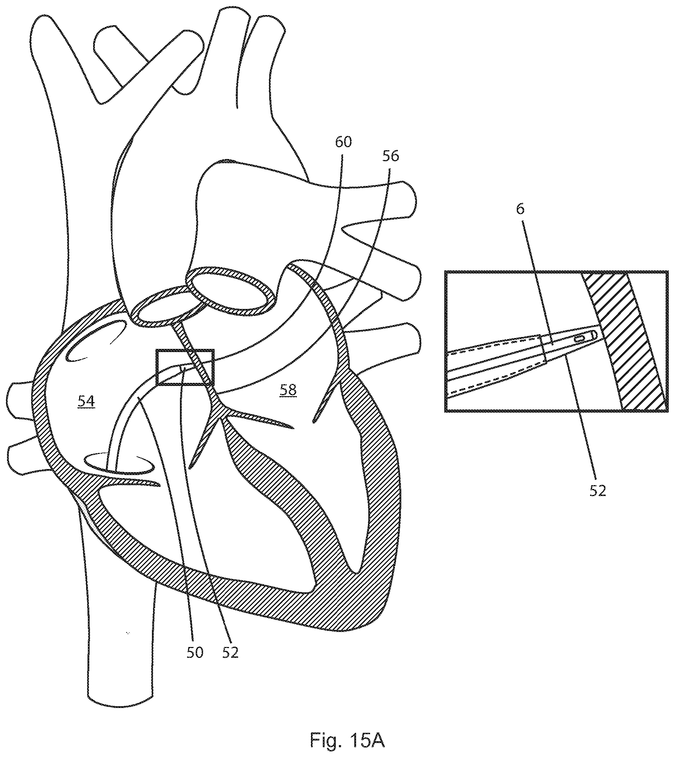

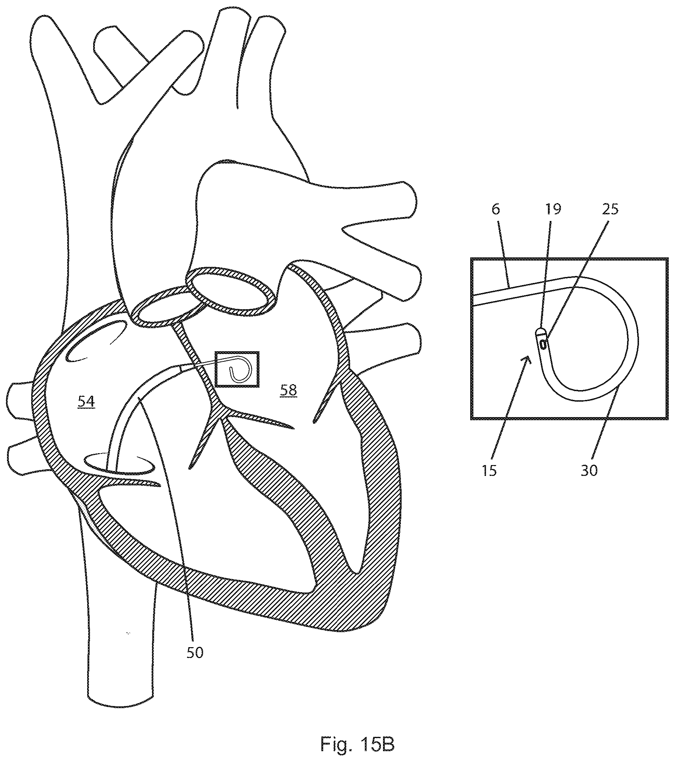

A further aspect of the disclosure is an exemplary and unclaimed method of creating a transseptal puncture. With reference now to Figs. 15A and 15B, an embodiment of this method aspect may comprise the steps of: (i) introducing an electrosurgical device 20 into a body of a patient, the electrosurgical device 20 comprising: an elongate member 6 having a distal region 24 and a proximal region 22 (Fig. 1), an energy delivery device 15 proximate to the distal region capable of cutting material, and a lumen 26 and apertures 25 operable to be in communication with a pressure sensing mechanism (not shown) for determining pressure in the body proximate to the distal region 24; (ii) positioning the energy delivery device 15 at a first desired location in the patient's body adjacent material to be cut; (iii) delivering energy using the energy delivery device 15 to cut said material; and (iv) measuring pressure in the body using the pressure sensing mechanism in order to determine the position of the electrosurgical device 20 at least one of before and after step (iii). In some embodiments of this aspect, step (ii) comprises delivering fluid (e.g. contrast fluid) for imaging at the first desired location in the patient's body.

Some embodiments of the method further comprise a step of (v) advancing the device to a second desired location. In certain embodiments of this aspect, the medical device comprises at least one radiopaque marker 5 and step (v) comprises monitoring at least one of said radiopaque markers 5. Some embodiments of the method comprise a step (vi) of measuring pressure at the second location. The medical device may comprise at least one radiopaque marker 5 and step (vi) may be performed after confirming the position of the pressure sensing mechanism at the second location using said radiopaque markers.

For some embodiments, step (i) comprises introducing the device into the patient's vasculature (and/or other body lumens). The step of introducing the device into the patient's vasculature may comprise inserting the device 20 into a dilator 52 and a guiding sheath 50 positioned in the patient's vasculature. In certain embodiments, the device 20 and at least one of the dilator 52 and sheath 50 each comprise a radiopaque marking and step (ii) comprises aligning the radiopaque markings to aid in positioning the device. For certain alternative embodiments of the method, step (v) comprises advancing the dilator 52 and the sheath 50 into the second location together over the spatially fixed electrosurgical device 20. In other alternative embodiments, step (v) comprises advancing the dilator, sheath and medical device all together into the second location.

In certain embodiments of this method aspect, the material is tissue located on an atrial septum 56 of a heart. Further, the region of tissue may be the fossa ovalis 60 of a heart. In such a case, the pressure measured at the first location is the blood pressure in the right atrium 54 and the pressure measured at the second location is the blood pressure in the left atrium 58.

In some alternative embodiments, the method further includes delivering imaging fluid that is visible using an imaging system in order to confirm the position of the electrosurgical device 20 at the second desired location.

In certain embodiments of the method, the medical device, dilator, and sheath are introduced into the heart via the inferior vena (Figs. 15A and 15B). In alternative embodiments, the heart is accessed from the superior vena cava (not shown in figures). Further details regarding superior and inferior approaches to the heart may be found in U.S. Patent Applications 13/113,326 (filed on May 23rd, 2011), and 11/265,304 (filed on November 3rd, 2005).

Thus, as described hereinabove, embodiments of the present invention include an electrosurgical device that comprises: an electrically conductive elongate member for traversing body vasculature configured and operable to allow energy to flow through the wall of the elongate member; a hollow lumen defined by the elongate member with one or more apertures defined by a wall of the elongate member at or near its distal end; and an energy delivery device in electrical communication with the elongate member (e.g. with the wall defining the lumen) at or about the distal end of the member. The energy delivery device has an electrode for delivering energy and a thermal shield is positioned between the electrode and the elongate member for protecting portions of the device from heat associated with the delivery of energy to tissue via the electrode. Method aspects of the present disclosure include using the electrosurgical device to traverse body vasculature of a patient and to deliver both fluid as well as electrical energy from or about its distal end.

The embodiments of the invention described above are intended to be exemplary only. The scope of the invention is therefore intended to be limited solely by the scope of the appended claims.

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims. In addition, citation or identification of any reference in this application shall not be construed as an admission that such reference is available as prior art to the present invention.

## Claims

1. An electrosurgical device (20) configured to apply electrical energy to cut through tissue in a region of a patient's body, said electrosurgical device (20) having
an elongate member (6) including an electrically conductive wall for delivering electrical energy therethrough, the elongate member (6) defining a lumen (26) that is in fluid communication with at least one distal aperture (25), said electrosurgical device (20) further comprising
an energy delivery device (15) including a proximal portion which is comprised of a metallic material in electrical communication with the electrically conductive wall of the elongate member (6), an electrode (19) which is comprised of a metal for delivering the electrical energy to a tissue, and an intermediate portion (18) which is metallic and between the proximal portion and the electrode (19), wherein a proximal end of the intermediate portion is proximate the proximal portion such that the intermediate portion cannot contact the elongate member (6); and
an electrically insulative thermal shield (3) between the electrode (19) and the elongate member (6) and **characterized in that**
the electrically insulative thermal shield (3) is cylindrical with a center bore and surrounds the intermediate portion (18) of the energy delivery device (15), wherein the intermediate portion (18) fills the center bore, and the energy delivery device (15) further blocks a distal end portion of the lumen (26).

2. The electrosurgical device (20) of claim 1, further comprising a layer of insulation (7) covering the elongate member (6), wherein at least a portion of the electrically insulative thermal shield (3) is positioned between the layer of insulation (7) and the electrode (19) for thermally protecting the layer of insulation (7).

3. The electrosurgical device (20) of claim 2, wherein a distal portion of the layer of insulation (7) is distal to an elongate member (6) distal end and wherein the distal portion of the layer of insulation (7) covers a proximal portion of the electrically insulative thermal shield (3).

4. The electrosurgical device (20) of claim 3, wherein the distal portion of the layer of insulation (7), at least in part, retains the electrically insulative thermal shield (3) in place.

5. The electrosurgical device (20) of claim 1, wherein the intermediate portion supports the electrically insulative thermal shield (3).

6. The electrosurgical device (20) of claim 1, wherein the elongate member (6) is flexible.

7. The electrosurgical device (20) of claim 1, wherein an outer diameter of_the intermediate portion and an outer diameter of the proximal portion are substantially equal.

8. The electrosurgical device (20) of claim 1, wherein the electrically insulative thermal shield (3) is comprised of a ceramic material.

9. The electrosurgical device (20) of claim 1, wherein the proximal portion is an electrically conductive spacer (4) at least partially occluding a distal part of the lumen (26).

10. The electrosurgical device (20) of claim 1, wherein the intermediate portion is elongate.

11. The electrosurgical device (20) of claim 7, wherein an outer diameter of the electrically insulative thermal shield (3) and an outer diameter of the elongate member (6) are substantially equal.

12. The electrosurgical device (20) of claim 1, wherein the electrically conductive wall has a helical configuration.

13. The electrosurgical device (20) of claim 1, wherein the elongate member (6) defines eight distal apertures (25).

14. The electrosurgical device (20) of claim 13, wherein the distal apertures (25) have a circular shape.

## Patentansprüche

1. Elektrochirurgische Vorrichtung (20), die dazu konfiguriert ist, elektrische Energie anzuwenden, um durch Gewebe in einem Bereich des Körpers eines Patienten zu schneiden, wobei die elektrochirurgische Vorrichtung (20) aufweist:
ein längliches Element (6), das eine elektrisch leitende Wand zum Liefern von elektrischer Energie dadurch beinhaltet, wobei das längliche Element (6) ein Lumen (26) definiert, das in Fluidverbindung mit mindestens einer distalen Öffnung (25) steht, wobei die elektrochirurgische Vorrichtung (20) ferner umfasst:
eine Energieliefervorrichtung (15), die einen proximalen Teil, der aus einem metallischen Material in elektrischer Verbindung mit der elektrisch leitenden Wand des länglichen Elements (6) besteht, eine Elektrode (19), die aus einem Metall zum Liefern der elektrischen Energie an ein Gewebe besteht, und einen Zwischenteil (18), der metallisch ist und sich zwischen dem proximalen Teil und der Elektrode (19) befindet, beinhaltet, wobei ein proximales Ende des Zwischenteils nahe dem proximalen Teil ist, sodass der Zwischenteil das längliche Element (6) nicht berühren kann; und
ein elektrisch isolierendes Hitzeschild (3) zwischen der Elektrode (19) und dem länglichen Element (6) und **dadurch gekennzeichnet, dass** das elektrisch isolierende Hitzeschild (3) zylindrisch mit einer mittleren Bohrung ist und den Zwischenteil (18) der Energieliefervorrichtung (15) umgibt, wobei der Zwischenteil (18) die mittlere Bohrung füllt und die Energieliefervorrichtung (15) ferner einen distalen Endteil des Lumens (26) blockiert.

2. Elektrochirurgische Vorrichtung (20) nach Anspruch 1, ferner umfassend eine Isolierschicht (7), die das längliche Element (6) bedeckt, wobei mindestens ein Teil des elektrisch isolierenden Hitzeschilds (3) zwischen der Isolierschicht (7) und der Elektrode (19) zum thermischen Schützen der Isolierschicht (7) positioniert ist.

3. Elektrochirurgische Vorrichtung (20) nach Anspruch 2, wobei ein distaler Teil der Isolierschicht (7) distal zu einem distalen Ende des länglichen Elements (6) ist und wobei der distale Teil der Isolierschicht (7) einen proximalen Teil des elektrisch isolierenden Hitzeschilds (3) bedeckt.

4. Elektrochirurgische Vorrichtung (20) nach Anspruch 3, wobei der distale Teil der Isolierschicht (7) zumindest teilweise das elektrisch isolierende Hitzeschild (3) an Ort und Stelle hält.

5. Elektrochirurgische Vorrichtung (20) nach Anspruch 1, wobei der Zwischenteil das elektrisch isolierende Hitzeschild (3) stützt.

6. Elektrochirurgische Vorrichtung (20) nach Anspruch 1, wobei das längliche Element (6) flexibel ist.

7. Elektrochirurgische Vorrichtung (20) nach Anspruch 1, wobei ein Außendurchmesser des Zwischenteils und ein Außendurchmesser des proximalen Teils im Wesentlichen gleich sind.

8. Elektrochirurgische Vorrichtung (20) nach Anspruch 1, wobei das elektrisch isolierende Hitzeschild (3) aus einem Keramikmaterial besteht.

9. Elektrochirurgische Vorrichtung (20) nach Anspruch 1, wobei der proximale Teil ein elektrisch leitender Abstandhalter (4) ist, der einen distalen Teil des Lumens (26) zumindest teilweise verdeckt.

10. Elektrochirurgische Vorrichtung (20) nach Anspruch 1, wobei der Zwischenteil länglich ist.

11. Elektrochirurgische Vorrichtung (20) nach Anspruch 7, wobei ein Außendurchmesser des elektrisch isolierenden Hitzeschilds (3) und ein Außendurchmesser des länglichen Elements (6) im Wesentlichen gleich sind.

12. Elektrochirurgische Vorrichtung (20) nach Anspruch 1, wobei die elektrisch leitende Wand eine schraubenförmige Konfiguration aufweist.

13. Elektrochirurgische Vorrichtung (20) nach Anspruch 1, wobei das längliche Element (6) acht distale Öffnungen (25) definiert.

14. Elektrochirurgische Vorrichtung (20) nach Anspruch 13, wobei die distalen Öffnungen (25) eine Kreisform aufweisen.

## Revendications

1. Dispositif électrochirurgical (20) configuré pour appliquer de l'énergie électrique afin de pratiquer une coupe au travers d'un tissu dans une région du corps d'un patient, ledit dispositif électrochirurgical (20) comportant :
un élément allongé (6) incluant une paroi électriquement conductrice pour délivrer de l'énergie électrique au travers, l'élément allongé (6) définissant une lumière (26) qui est en communication fluidique avec au moins une ouverture distale (25), ledit dispositif électrochirurgical (20) comprenant en outre :
un dispositif de délivrance d'énergie (15) incluant une section proximale qui est constituée en un matériau métallique en communication électrique avec la paroi électriquement conductrice de l'élément allongé (6), une électrode (19) qui est constituée en un métal pour délivrer l'énergie électrique à un tissu et une section intermédiaire (18) qui est métallique et qui est située entre la section proximale et l'électrode (19), dans lequel une extrémité proximale de la section intermédiaire est à proximité de la section proximale de telle sorte que la section intermédiaire ne puisse pas entrer en contact avec l'élément allongé (6) ; et
une protection thermique électriquement isolante (3) entre l'électrode (19) et l'élément allongé (6), et **caractérisé en ce que** : la protection thermique électriquement isolante (3) est cylindrique, est munie d'un alésage central et entoure la section intermédiaire (18) du dispositif de délivrance d'énergie (15), et dans lequel la section intermédiaire (18) remplit l'alésage central et le dispositif de délivrance d'énergie (15) bloque en outre une section d'extrémité distale de la lumière (26).

2. Dispositif électrochirurgical (20) selon la revendication 1, comprenant en outre une couche d'isolation (7) qui recouvre l'élément allongé (6), dans lequel au moins une section de la protection thermique électriquement isolante (3) est positionnée entre la couche d'isolation (7) et l'électrode (19) pour protéger thermiquement la couche d'isolation (7).

3. Dispositif électrochirurgical (20) selon la revendication 2, dans lequel une section distale de la couche d'isolation (7) est distale par rapport à une extrémité distale de l'élément allongé (6) et dans lequel la section distale de la couche d'isolation (7) recouvre une section proximale de la protection thermique électriquement isolante (3).

4. Dispositif électrochirurgical (20) selon la revendication 3, dans lequel la section distale de la couche d'isolation (7) retient en place, au moins en partie, la protection thermique électriquement isolante (3).

5. Dispositif électrochirurgical (20) selon la revendication 1, dans lequel la section intermédiaire supporte la protection thermique électriquement isolante (3).

6. Dispositif électrochirurgical (20) selon la revendication 1, dans lequel l'élément allongé (6) est souple.

7. Dispositif électrochirurgical (20) selon la revendication 1, dans lequel un diamètre externe de la section intermédiaire et un diamètre externe de la section proximale sont sensiblement égaux.

8. Dispositif électrochirurgical (20) selon la revendication 1, dans lequel la protection thermique électriquement isolante (3) est constituée en un matériau de céramique.

9. Dispositif électrochirurgical (20) selon la revendication 1, dans lequel la section proximale est un espaceur électriquement conducteur (4) qui ferme au moins partiellement une partie distale de la lumière (26).

10. Dispositif électrochirurgical (20) selon la revendication 1, dans lequel la section intermédiaire est allongée.

11. Dispositif électrochirurgical (20) selon la revendication 7, dans lequel un diamètre externe de la protection thermique électriquement isolante (3) et un diamètre externe de l'élément allongé (6) sont sensiblement égaux.

12. Dispositif électrochirurgical (20) selon la revendication 1, dans lequel la paroi électriquement conductrice présente une configuration en hélice.

13. Dispositif électrochirurgical (20) selon la revendication 1, dans lequel l'élément allongé (6) définit huit ouvertures distales (25).

14. Dispositif électrochirurgical (20) selon la revendication 13, dans lequel les ouvertures distales (25) présentent une forme circulaire.
